# EUROPEAN PATENT APPLICATION

(11) **EP 4 563 089 A1**
(43) Date of publication of application: **04.06.2025**
(21) Application number: 23307066.3
(22) Date of filing: 28.11.2023
(51) Int. Cl.: A61B 8/08, G06T 7/00, G06T 7/60, G16H 50/20, A61B 8/00

(54) **TRACKING ULTRASOUND IMAGES**

(71) Applicant: Koninklijke Philips N.V., 5656 AG Eindhoven (NL)
(72) Inventor: SCHMIDT-RICHBERG, Alexander, 5656 EINDHOVEN (NL); ORASANU, Eliza, 5656 EINDHOVEN (NL); LORENZ, Cristian, 5656 EINDHOVEN (NL); FRANZ, Astrid, 5656 EINDHOVEN (NL); RAYNAUD, Caroline, 5656 EINDHOVEN (NL); DE CRAENE, Mathieu, 5656 EINDHOVEN (NL); CIOFOLO-VEIT, Cybele, 5656 EINDHOVEN (NL); DUFOUR, Cécile, 5656 EINDHOVEN (NL)
(74) Representative: Philips Intellectual Property & Standards

(57) **Abstract**

A mechanism for tracking one or more ultrasound images for one or more anatomical views. An ultrasound image is processed to identify the anatomical view represented by the ultrasound image. The ultrasound image is compared to a set of one or more historic ultrasound images, providing the same view, to determine whether or not the ultrasound image provides a higher quality depiction of the anatomical view. If the ultrasound image provides a higher quality depiction, view tracking information is updated to replace reference to a historic ultrasound image (in the set) with the ultrasound image.

## Description

### FIELD OF THE INVENTION

The present invention relates to the field of medical imaging, and in particular, to ultrasound medical imaging.

### BACKGROUND OF THE INVENTION

There is an ongoing interest in the field of medicine for using medical imaging techniques to analyze and/or assess the condition of a subject or patient. Ultrasound imaging is a particularly useful method for performing medical imaging, as it can be performed using relatively low-cost and widely available imaging systems and has no known adverse effect on the imaged subject.

In the field of ultrasound imaging, it is common to produce ultrasound images that provide desired anatomical views of the subject. This is essential for accurate determination of certain biometric measurements that are commonly required for assessing the condition of a subject. For instance, in fetal ultrasound imaging, there are a number of predetermined biometric measures (such as head circumference, femur length and so on) that are taken to assess growth and development of the fetus.

To facilitate measurement of such biometric measurements, the operator of the ultrasound imaging system is therefore required to manipulate the ultrasound imaging system to produce at least one ultrasound image for each desired anatomical view. Typically, when a desired anatomical view is displayed at a user interface, the operator will manually identify the ultrasound image (e.g., using a "freeze" button) and perform the relevant biometric measurement(s) using the identified ultrasound image.

There is an ongoing desire to facilitate more accurate identification of ultrasound images that provide a desired anatomical view, e.g., for improved and more accurate performance of a biometric measurement using the ultrasound images.

### SUMMARY OF THE INVENTION

The invention is defined by the independent claims. The dependent claims define advantageous embodiments.

According to examples in accordance with an aspect of the invention, there is provided a computer-implemented method for tracking one or more ultrasound images of a subject.

The computer-implemented method comprises: receiving an ultrasound image of the subject; receiving view tracking information identifying, for each of one or more predetermined anatomical views, a respective set of one or more historic ultrasound images of the subject associated with the predetermined anatomical view; processing the ultrasound image to predict an anatomical view of the ultrasound image; responsive to the predicted anatomical view of the ultrasound image matching any of the predetermined anatomical views: predicting whether or not the ultrasound image is of a higher quality than at least one historic ultrasound image in the set of one or more historic ultrasound images associated with the predetermined anatomical view matching the anatomical view of the ultrasound image; and responsive to predicting that the ultrasound image is of a higher quality than at least one historic ultrasound image, modifying the view tracking information to further identify the ultrasound image and no longer a historic ultrasound image predicted to have a lower quality than the ultrasound image, in the set of one or more historic ultrasound images associated with the predetermined anatomical view matching the anatomical view of the ultrasound image.

The present disclosure proposes to update view tracking information with reference to a (new) ultrasound image, if the ultrasound image is determined to provide a higher quality representation of a desired anatomical view than a pre-existing or known ultrasound image.

In particular, embodiments effectively propose to track the N best (effectively, highest quality) ultrasound images for each of one or more different anatomical views. This advantageously makes it easy for a clinician to assess if a suitable ultrasound image for an anatomical view have already been acquired or, whether further ultrasound imaging is required. The proposed approach thereby credibly assists a clinician or operator in performing a technical process of obtaining at least one high-quality ultrasound image for each of a plurality of anatomical views.

In the context of the present disclosure, a historic ultrasound image is an ultrasound image of the subject that was previously generated or otherwise made available. In particular, a historic ultrasound image may be another ultrasound image that was generated during a same image capturing process as the ultrasound image, e.g., during a same imaging examination of the subject. In some instances, each historic ultrasound image and the ultrasound image may form frames or images of a same sequence of ultrasound images captured during a single image capturing process, e.g., form part of a same ultrasound image stream produced by an ultrasound imaging device.

In the context of the present disclosure, an anatomical view is a predefined label for a field-of-view of a subject, which should contain a view (from a particular perspective or viewpoint) of a set of one or more anatomical objects.

The one or more predetermined anatomical views may comprise a plurality of predetermined anatomical views. It is recognized that a clinician will often wish or desire to look at multiple anatomical views when making clinical decisions, such that the overall understanding and information available to a clinician is increased if multiple predetermined anatomical views are tracked. This makes the tracking of the ultrasound image(s) more clinically relevant.

The computer-implemented method may further comprise controlling a user interface to provide a visual representation of the ultrasound image and each historic ultrasound image.

This helps guide a clinician or operator of an ultrasound imaging system as to whether a high-quality ultrasound image, or enough high-quality ultrasound images for each anatomical view has/have been obtained.

In some examples, the visual representation of each historic ultrasound image, provided by the user interface, is interactive.

In some examples, the computer-implemented method further comprises, responsive to a user interaction with the interactive visual representation of any historic ultrasound image, processing the historic ultrasound image associated with the interacted visual representation to determine one or more biometric measurements of one or more anatomical features represented in the historic ultrasound image. This facilitates the automated performance of a technically difficult task, allowing information about the subject to be automatically and accurately generated. In particular, as the historic ultrasound image(s) will be of a high quality, generating the biometric measurement by an interaction with said historic ultrasound image will ensure that accurate biometric measurements are produced.

The computer-implemented method may further comprise controlling the user interface to display the determined one or more biometric measurements. This provides the clinician or operator with useful information about the state of the subject (undergoing ultrasound imaging).

In some examples, each set of one or more historic ultrasound images comprises at least two historic ultrasound images.

In some examples, the step of processing the ultrasound image to predict the anatomical view of the ultrasound image comprises processing the ultrasound image using one or more classification algorithms. In some examples, the one or more classification algorithms comprises one or more machine-learning classification algorithms. The one or more machine-learning classification algorithms may comprise one or more you-only-look-once object detection algorithms.

The step of predicting whether or not the ultrasound image is of a higher quality than at least one historic ultrasound image comprises performing a pairwise comparison of the ultrasound image to at least one of the one or more historic ultrasound images. This reduces or avoids a reliance upon a subjective, absolute quality value determination procedure for determining the quality of different ultrasound images.

This approach recognizes that automated quality scoring (to produce an absolute quality value) is misleading or inaccurate, as a quality score will be highly subjective, particularly when comparing ultrasound images of different patients/examinations. Moreover, it is recognized that any such quality score will dependent on the whole sequence, not only on the ultrasound image individually. For example, sometimes a better ultrasound image can be acquired by slightly moving the probe. In other cases, the target structure would be shadowed when moving the ultrasound image, such that a better ultrasound image cannot be acquired. Thus, a highest (possible) quality ultrasound image may, if processed using an absolute scoring method, be assigned a low quality score when, in practice, it is the highest quality available for the subject.

The proposed approach of using pairwise comparisons avoids or mitigates this problem.

In some examples, the pairwise comparison is performed using a machine-learning comparative classification algorithm.

In other examples, the step of predicting whether or not the ultrasound image is of a higher quality than at least one historic ultrasound image comprises: determining a quality metric for the ultrasound image and each historic ultrasound image in the set of one or more ultrasound images associated with the predetermined anatomical view matching the anatomical view of the ultrasound image; and comparing the quality metric of the ultrasound image to the quality metric of at least the lowest quality historic ultrasound image to predict whether or not the ultrasound image is of a higher quality than at least one historic ultrasound image in said set.

This approach effectively makes use of a mechanism for performing absolute scoring on the ultrasound image (and each historic ultrasound image). This allows the use of existing techniques and processing for scoring or measuring a quality of an ultrasound image. There is also provided a computer program product or non-transitory storage medium comprising computer program code or instructions which, when executed on a computing device having a processing system, cause the processing system to perform all of the steps of any herein disclosed method.

There is also provided a processing system tracking one or more ultrasound images of a subject, the processing system being configured to perform any of the herein disclosed methods.

There is also provided an ultrasound imaging system comprising: the processing system; and an ultrasound transducer system configured to generate the ultrasound image and each historic ultrasound image.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an ultrasound imaging system;
Fig. 2 is a flowchart illustrating a proposed method;
Fig. 3 illustrates a display produced by a proposed method; and
Fig. 4 is a flowchart illustrating a further method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for tracking one or more ultrasound images for one or more anatomical views. An ultrasound image is processed to identify the anatomical view represented by the ultrasound image. The ultrasound image is compared to a set of one or more historic ultrasound images, providing the same view, to determine whether or not the ultrasound image provides a higher quality depiction of the anatomical view. If the ultrasound image provides a higher quality depiction, view tracking information is updated to replace reference to a historic ultrasound image (in the set) with the ultrasound image.

A number of proposed embodiments make use of a machine-learning algorithm to performing the processing of some data. A machine-learning algorithm is any self-training algorithm that processes input data in order to produce or predict output data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g., the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries.

For some machine-learning algorithms, such as a neural network, training is performed by applying an initialized machine-learning algorithm to each input data entry to generate predicted output data entries. An error (being a value produced by a loss function) between the predicted output data entries and corresponding training output data entries is used to modify the machine-learning algorithm. This process can be repeated until the error converges, and the predicted output data entries are sufficiently similar (e.g., ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The content of the training input data entries and the training output data entries will be responsive to the data to be processed and the desired data to be predicted, as will be readily apparent to the skilled person.

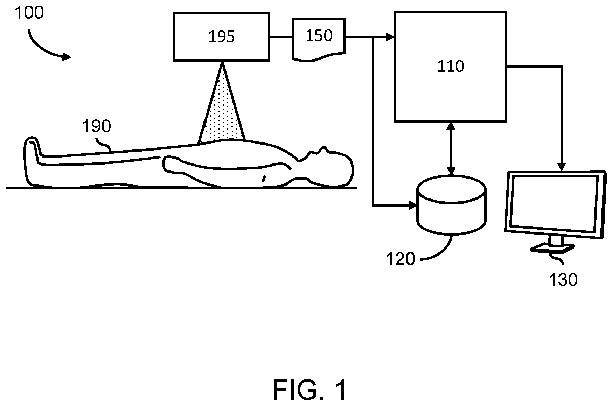

Fig. 1 conceptually illustrates an ultrasound imaging system 100 in which embodiments can be implemented, for the sake of improved contextual understanding.

An ultrasound transducer system 195 is able to generate ultrasound images 150 of a subject 190 using an ultrasound imaging process. Techniques for operating an ultrasound transducer system and generating ultrasound images 150 are well-established in the art, and are not herein described for the sake of conciseness. The ultrasound transducer may be in immediate contact with the subject 190 when acquiring ultrasound images.

Any ultrasound image 150 generated by the ultrasound transducer system 195 may be passed to a processing system 110 for further processing and/or a memory 120 or storage unit. The processing system 110 may be configured to receive or retrieve any ultrasound image(s) stored in the memory 120.

The processing system 110 may be configured to control a user interface 130 to display any ultrasound image. In this context, controlling a user interface 130 to display an ultrasound image comprises providing a display signal that causes the user interface 130 to provide a visual representation of the ultrasound image. Typically, the processing system 110 will be configured to control the user interface 130 to display the ultrasound image that was most recently obtained by the ultrasound transducer system 195.

It is possible to process an ultrasound image in order to identify or predict an anatomical view provided by the ultrasound image. By way of example, the European Patent Applications having publication numbers EP 4,041,086 A1 and EP 4,091,550 A1 disclose approaches for identifying a view provided by the ultrasound image. In particular, view recognition may make use of one or more machine-learning algorithms to process the ultrasound image and determine or predict which view is most likely represented by the ultrasound image.

It is also possible to process an ultrasound image in order to identify or predict a quality (e.g., a quality score/metric) of the ultrasound image. Various approaches for analyzing a medical image to determine or predict a quality of the medical image are known in the art, such as the approaches disclosed by European Patent Applications having publication numbers EP 4,075,613 A1; EP 4,241,239 A1; and/or EP 3,923,293 A1. Other approaches for predicting a quality or quality score/metric will be readily apparent to the skilled person, e.g., as set out by Dobbins III, James T. "Image quality metrics for digital systems." Handbook of medical imaging 1 (2000): 161-222. Examples of suitable quality scores/metrics include measurements of: (inherent) contrast; noise; artifacts; signal-to-noise ratio; MTF and so on. A preferred approach for determining a quality of a medical image will be provided later in this disclosure.

Thus, it is possible to process an ultrasound image to generate metadata or information that identifies an anatomical view of the historic ultrasound image and/or a quality of the historic ultrasound image.

The present invention proposes an approach for selective provision or tracking of one or more ultrasound images that aid a clinician or operator of the ultrasound transducer system to perform a clinical task, such as subject analysis, diagnosis and/or assessment.

More particularly, it has been recognized that a clinician looking to perform an analysis of a particular anatomical element will benefit from a predetermined set of one or more views (so-called "standard views") of the anatomical element to perform effective analysis. For instance, when wishing to assess the condition of a fetal head, a clinician may desire: a transathalamic (TT) plane view of the fetal head; a transcerebellar (TC) plane view of the fetal head and a transventricular (TV) plane view of the fetal head.

The present disclosure proposes a technique for tracking one or more high quality ultrasound images for a subject. More particularly, the present invention proposes a technique for tracking, for each of one or more predetermined anatomical views, high quality ultrasound images that provide the anatomical view(s).

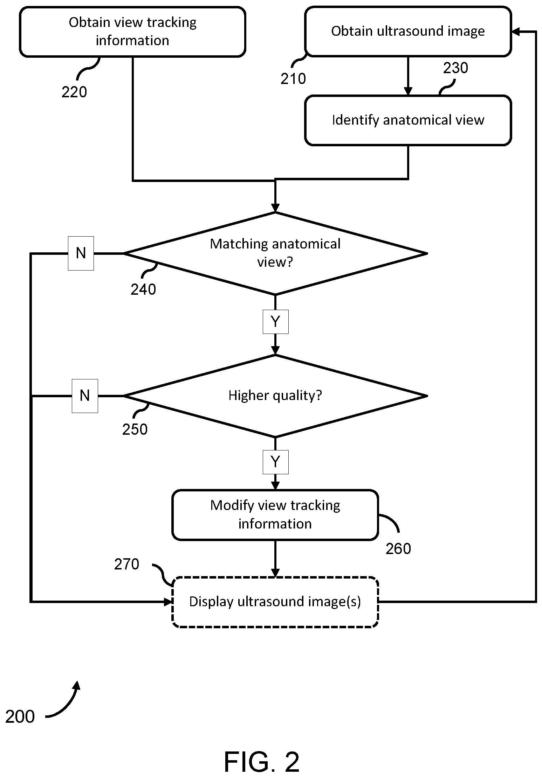

Fig. 2 illustrates a proposed computer-implemented method 200 for tracking one or more ultrasound images for the subject, including an optional process for displaying one or more ultrasound images at a user interface. The computer-implemented method 200 may be performed by the processing system 110 previously described.

The method 200 comprises a step 210 of receiving or obtaining an ultrasound image of the subject, e.g., for display by the user interface. The ultrasound image may, for instance, be received or obtained directly from the ultrasound transducer system 195 or from the memory 120.

In particular, the ultrasound image may be a most recently received, obtained, generated or otherwise made available ultrasound image for the subject. This facilitates live (or near-live) tracking of ultrasound images.

The method 200 also comprises a step 220 of receiving or obtaining view tracking information. The view tracking information identifies, for each of one or more predetermined anatomical views (e.g., a plurality of predetermined anatomical views), a respective set of one or more historic ultrasound images of the subject associated with the predetermined anatomical view.

The number of one or more historic ultrasound images in each set may be limited to a maximum (predetermined) number N. The value of N may, for instance, be no greater than 5, e.g., 2, e.g., 1. Each historic ultrasound image may, for instance, comprise an ultrasound image that is captured in a same (single) image capturing session or imaging examination that produced/produces the ultrasound image obtained in step 210. Put another way, each historic ultrasound image may be a frame or ultrasound image from a same sequence of ultrasound images as the ultrasound image obtained in step 210.

Thus, in a working example, the ultrasound image (obtained in step 210) may be a most recently available ultrasound image from a stream of ultrasound images, with each historic ultrasound image being a previous ultrasound image in the (same) stream of ultrasound images.

The view tracking information may, for instance, be received, obtained or retrieved from the memory 120 or a buffer of the processing system 110.

The view tracking information may, for instance, comprise each historic ultrasound image, e.g., with an accompanying instance of metadata for each historic ultrasound image identifying the anatomical view provided by the historic ultrasound image. Such metadata) may be obtained, for instance, from the memory 120. The metadata for each historic ultrasound image may have been produced, for instance, by the processing system 110 or by a separate, different processing system (e.g., a cloud-based processing system).

In an alternative example, the view tracking information comprises an array or vector of one or more identifiers, each of which identifies a respective, relevant historic ultrasound image. Each identifier may, for instance, take the form of a pointer or near-unique code for the historic ultrasound image. A position of the identifier within the array or vector may indicate the corresponding anatomical view. In other examples, each identifier is accompanied with anatomical view data identifying the anatomical view of the corresponding historic ultrasound image.

The method 200 also comprises a step 230 of processing the ultrasound image to predict the anatomical view of the ultrasound image. A number of approaches for identifying an anatomical view of an ultrasound image have been previously described.

In some examples, step 230 comprises processing the ultrasound image using one or more classification algorithms. The one or more classification algorithms may, for instance, comprises one or more machine-learning classification algorithms. In particular, the one or more machine-learning classification algorithms may comprise one or more you-only-look-once object (YOLO) detection algorithms.

As a working example, the ultrasound image may be processed using one or more object detection algorithms (e.g., YOLO detection algorithms) on the ultrasound image to detect any anatomical structures represented in the anatomical image. A confidence value may be estimated for each detected structure. One or more identification rules can then be applied on the confidence values to determine the most probable view. This approach appreciates that each anatomical view is likely to contain a different set of anatomical features or structures. Accordingly, it is possible to identify the anatomical structures represented by an ultrasound image, and determine or predict the anatomical view provided by the ultrasound image accordingly.

In an alternative example, a machine-learning algorithm may be trained to receive, as input, the ultrasound image and provide, as output, a predicted anatomical view provided by the ultrasound image. Thus, the machine-learning algorithm may be directly trained to predict the anatomical view of a (processed) ultrasound image.

The method 200 also comprises a determination step 240, which determines whether or not the predicted anatomical view (produced in step 230) matches any of the one or more predetermined anatomical views (in the view tracking information). This can be readily achieved by determining whether or not the predicted anatomical view matches one of the one or more predetermined anatomical views.

Responsive to a positive determination in step 240, the method performs a step 250 of predicting whether or not the ultrasound image is of a higher quality than at least one historic ultrasound image in the relevant set of one or more historic ultrasound images. In this context, the "relevant set" is the set of one or more historic ultrasound images having the one or more historic ultrasound images associated with the predetermined anatomical view that matches (i.e., is the same as) the anatomical view of the ultrasound image.

Responsive to a positive determination in step 250, the method 200 performs a step 260 of modifying the view tracking information to further identify the ultrasound image and no longer identify one of the historic ultrasound images. The historic ultrasound image that is no longer identified is one of the historic ultrasound images that belongs to the relevant set and for which the ultrasound image is a higher quality than said historic ultrasound image. This effectively provides a mechanism for updating the view tracking information to continually track the ultrasound image(s) that provide the highest quality representation of the predetermined anatomical views.

Responsive to a negative determination in either step 240 or 250, then step 260 is not performed.

The method 200 may comprise a step 270 of controlling a user interface to provide a visual representation of the ultrasound image and each historic ultrasound image (identified in the view tracking information). Step 270 may, for instance, comprise obtaining each historic ultrasound image (e.g., from the memory 120) and controlling the user interface to provide a visual representation of the ultrasound image and each historic ultrasound image.

Thus, step 270 effectively comprises displaying the ultrasound image and each historic ultrasound image (identified in the view tracking information).

Where there are a plurality of historic ultrasound images identified in the view tracking information, step 270 may be modified to comprise controlling the user interface to provide controlling a user interface to provide a visual representation of the ultrasound image and a subset (i.e., not all) of the historic ultrasound image(s) identified in the view tracking information. For instance, in some examples where each set of one or more historic ultrasound images (identified in the tracking information) comprises a plurality of historic ultrasound images, only a subset (i.e., not all) historic ultrasound images are visually represented at the user interface. The selection or identification of the subset may be responsive to a user selection.

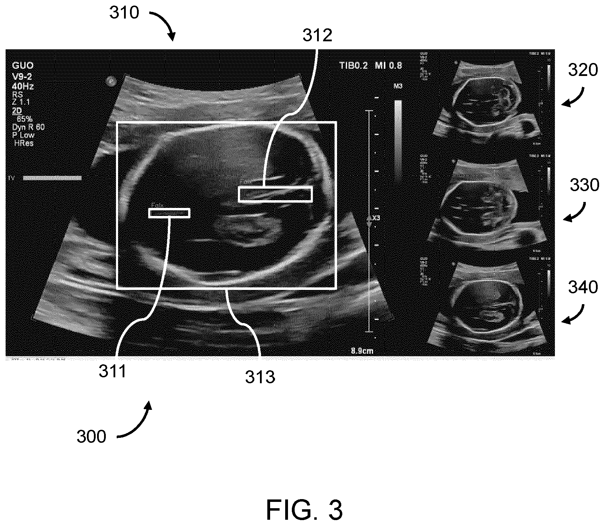

Fig. 3 illustrates an example display 300 provided by a user interface that has controlled to provide a visual representation of the ultrasound image 310 and each historic ultrasound image 320, 330, 340. In this example, each set of one or more historic ultrasound images associated with each predetermined anatomical view comprises a single historic ultrasound image.

Fig. 3 also conceptually illustrates, for the sake of illustrative clarity only, bounding boxes 311, 312 that represent anatomical elements in the ultrasound image and a bounding box 313 representing an anatomical view provided by the ultrasound image.

Turning back to Fig. 2, the method 200 may be iteratively repeated. In this way, the best N ultrasound images for each predetermined anatomical view can be tracked over time. The value of N may be any positive integer, e.g., 1, 2, 3, 5 or 10.

One approach for performing step 250 is to generate a quality metric for each ultrasound image and each historic ultrasound image (in the relevant set), e.g., using a previously described technique. The quality metrics can then be compared to determine which ultrasound image has a higher quality. This approach effectively makes use of a mechanism for performing absolute scoring on the ultrasound image (and each historic ultrasound image).

In this technique, step 250 may comprise comparing the ultrasound image to (only) the lowest quality historic ultrasound image in the relevant set. The lowest quality historic ultrasound image can be identified from the quality metric of the historic ultrasound image(s). A comparison between the ultrasound image and a historic ultrasound image may comprise comparing the quality metrics to determine which ultrasound image is of a higher quality.

A preferred approach to performing step 250 is to perform a pairwise comparison of the ultrasound image to at least one historic ultrasound image in the relevant set. Thus, rather than individually assessing each ultrasound image to determine an absolute quality metric for comparison, the ultrasound images may be processed to determine a relative quality metric or identify a relatively higher quality one of the compared ultrasound images. This approach therefore effectively makes use of a mechanism for performing relative scoring or relative quality determination between the ultrasound image and at least one historic ultrasound image.

Thus, step 250 may comprise performing a relative comparison (i.e., a pairwise comparison) of quality between the ultrasound image and at least one historic ultrasound image. Thus, rather than attempting to establish an absolute quality of all (current and historic) ultrasound images, two ultrasound images may be compared relatively to one another.

In this technique, the relative or pairwise comparison can be trivially performed if the set of one or more historic ultrasound images comprises only a single historic ultrasound image. More particularly, step 250 may simply comprise performing a pairwise comparison between the ultrasound image and the single historic ultrasound image in the relevant set to identify whether or not the ultrasound image has a higher quality than at least one historic ultrasound image.

In a variation in which the one or more historic ultrasound images in the relevant set comprises a plurality of historic ultrasound images, the ultrasound image may be compared to each historic ultrasound image in the relevant set in turn, i.e., in sequence. The iterative comparisons may be stopped if a historic ultrasound image having a lower quality than the ultrasound image is identified. In this approach, step 260 comprises modifying the view tracking information to no longer identify the first identified historic ultrasound image having a lower quality than the ultrasound image (if any).

In another variation in which the one or more historic ultrasound images in the relevant set comprises a plurality of historic ultrasound images, the plurality of historic ultrasound images in the relevant set may be ordered in a sequence, e.g., effectively representing a hierarchy of the historic ultrasound image(s). The ultrasound image may be compared to each historic ultrasound image in the sequence (either in turn or simultaneously). The historic ultrasound image that is earliest in the sequence, amongst all the historic ultrasound image(s) that are determined to be of a lower quality than the ultrasound image (if any), is determined. If performed, step 260 correspondingly comprises modifying the view tracking information to no longer identify this determined historic ultrasound image. Of course, if no historic ultrasound image in the sequence is determined to be of a lower quality than the ultrasound image, then no historic ultrasound image will be determined in step 250, and step 260 will not be performed.

A number of approaches for performing a pairwise comparison between two ultrasound images (e.g., the ultrasound image and a historic ultrasound image) are hereafter described.

One approach to performing a pairwise comparison is to treat the pairwise comparison as a classification problem of two ultrasound images. In this approach, information derived from two ultrasound images is provided as input to a machine-learning comparative classification algorithm (e.g., a neural network). As a working example, the entirety of each ultrasound image may be provided as input to the machine-learning algorithm. The machine-learning algorithm may be appropriately trained to output a first value (e.g., +1) if a first ultrasound image has a higher quality and a second value (e.g., -1) if the second ultrasound image has a higher quality. Tracking the value output by the machine-learning algorithm thereby facilitates predicting which ultrasound image is of a higher quality.

Optionally, the machine-learning algorithm may be appropriately trained to output a third value (e.g., 0) for ultrasound images of equal quality. In such instances, step 240 may arbitrarily decide whether or not the ultrasound image is of the higher quality than a historic ultrasound image, e.g., perform a (pseudo)random selection or a predetermined selection (e.g., always choose the historic ultrasound image).

In this approach, the machine-learning algorithm can be appropriately trained by a loss function that penalizes a difference between an output value and a user-defined example value.

Another approach to performing a pairwise comparison is to treat the pairwise comparison as a grading problem, which can be solved (for example) using a Siamese network architecture. In this approach, each ultrasound image is run individually through the same machine-learning algorithm (with shared weights) to compute a respective quality score (i.e., thereby outputting two quality scores Q1 and Q2). To train the model, the quality scores are used in the loss (i.e., the network is trained such that Q1>Q2 or Ql <Q2, respectively).

Some embodiments recognize that ultrasound images (in particular, fetal ultrasound images) often have large and heterogeneous background regions, which contain little to no relevant information for assessing a quality of the ultrasound image, even in a pairwise comparison process.

One embodiment therefore additionally proposes to use focus regions - i.e., regions of interest - of the ultrasound image as the input to the machine-learning algorithm. It will be appreciated that focus regions will be specific to an anatomical view, and should contain representations of anatomical structures that are relevant for the quality decision of an ultrasound image of the anatomical view. For example, the Head, Cerebellum and Cisterna Magna regions are relevant for the grading of a TC anatomical view.

Thus, in some examples, step 250 may comprise identifying one or more regions of the ultrasound image that contain a representation of one or more anatomical objects. The one or more anatomical objects may be predetermined, e.g., responsive to the identified anatomical view in step 230. Region identification can be performed using an objection detection algorithm (such as a YOLO algorithm). If such an algorithm is used in step 230, then the algorithm does not need to be repeated in step 250.

Similarly, step 250 may comprise identifying one or more regions of the at least one historic ultrasound image (of the relevant set) that contain a representation of the one or more anatomical objects. A similar procedure to that previously disclosed may be employed to process the historic ultrasound image.

Step 250 may then comprise processing the identified regions of the ultrasound image and the historic ultrasound image, for example, using a machine-learning algorithm to predict whether or not the ultrasound image is of a higher quality than a historic ultrasound image in the set of one or more historic ultrasound images associated with the predetermined anatomical view matching the anatomical view of the ultrasound image.

Accordingly, the machine-learning algorithm used to process the regions of interest may comprise no less than R input channels, where R is the number of regions of interest. If a machine-learning algorithm is configured to simultaneously process information derived from two ultrasound images, then the machine-learning algorithm will comprise no fewer than 2R input channels.

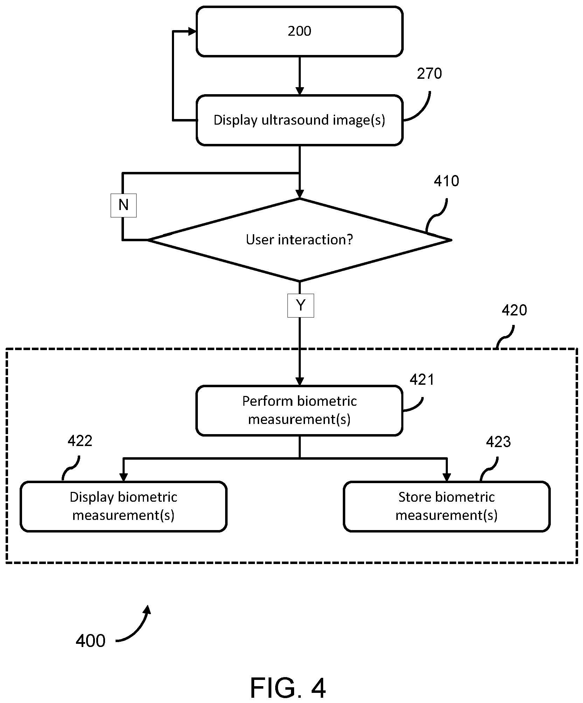

Fig. 4 illustrates a method 400 according to a further embodiment. The method 400 comprises performing any previously described method 200 for tracking ultrasound images of a subject.

The method also includes the previously described step 270 of displaying the obtained ultrasound image and each historic ultrasound image. In particular, step 270 comprises controlling a user interface to provide a visual representation of the ultrasound image and each historic ultrasound image.

The step 270 is configured such that the visual representation of each historic image is interactive. Precise mechanisms for making a visual representation interactive are well known in the art, and may depend upon the form of the user interface. For instance, the visual representation may be provided at a touch-sensitive user interface and an interaction may be a touch at the location of the visual representation. As another example, an interaction may be detected by monitoring the movement of a cursor on a display and registering a click (e.g., of a computer mouse or other interface element) representing an interaction with the visual representation.

It will be appreciated that method 200 any 270 may be iteratively repeated independently of any other procedure or steps performed by the method 400.

The method 400 comprises a step 410 of monitoring the user interface for any interaction with any interactive visual representation (of a historic ultrasound device). Thus, step 410 may comprise determining whether or not there is a user interaction with the interactive visual representation of any historic ultrasound image.

Responsive to a negative determination in step 410, the method 400 may hang or stall, e.g., at step 410.

Responsive to a positive determination in step 420, the method 400 may perform a further processing procedure 420 on the historic ultrasound image that is visually represented by (i.e., associated with) the interacted visual representation.

In the illustrated example, the further processing procedure 420 comprises processing the historic ultrasound image that is visually represented by (i.e., associated with) the interacted visual representation to determine or predict one or more biometric measurements of one or more anatomical features represented in said historic ultrasound image.

As the anatomical view of the historic ultrasound image is known, then the anatomical elements expected to be represented in the historic ultrasound image are known. Performing biometric measurements can therefore be achieved by performing an automated biometric measurement of one or more anatomical elements or structures expected to be in an ultrasound image.

Approaches for automated biometric measuring (also known as anatomical measuring) of ultrasound images are well-established in the art, for instance, as exemplified by the European Patent Applications having publication numbers EP 3,506,832 A1; and/or EP 3,742,973 A1.

As an example, a biometric measurement may be identified, for instance, by segmenting the historic ultrasound image, e.g., using an appropriately trained machine-learning algorithm and/or contouring mechanisms, and using an automated calipers or the like to determine the biometric measurement(s).

The method 400 may comprise a step 422 of controlling the user interface to provide a visual representation of the determined biometric measurement(s). Thus, step 421 may effectively comprise displaying the biometric measurement(s).

The method 400 may comprise a step 423 of storing the biometric measurement(s), e.g., in the memory or storage unit.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

Any such processor system may be integrated into an ultrasound imaging system. In particular, there may be provided an ultrasound imaging system comprising: the processing system; and an ultrasound transducer system configured to generate the ultrasound image and each historic ultrasound image. The ultrasound imaging system may further comprise a user interface, e.g., for displaying the ultrasound image and/or each historic ultrasound image.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

**1.** A computer-implemented method for tracking one or more ultrasound images of a subject, the computer-implemented method comprising:
receiving an ultrasound image of the subject;
receiving view tracking information identifying, for each of one or more predetermined anatomical views, a respective set of one or more historic ultrasound images of the subject associated with the predetermined anatomical view;
processing the ultrasound image to predict an anatomical view of the ultrasound image;
responsive to the predicted anatomical view of the ultrasound image matching any of the predetermined anatomical views:
predicting whether or not the ultrasound image is of a higher quality than at least one historic ultrasound image in the set of one or more historic ultrasound images associated with the predetermined anatomical view matching the anatomical view of the ultrasound image; and
responsive to predicting that the ultrasound image is of a higher quality than at least one historic ultrasound image, modifying the view tracking information to further identify the ultrasound image and no longer identify a historic ultrasound image predicted to have a lower quality than the ultrasound image, in the set of one or more historic ultrasound images associated with the predetermined anatomical view matching the anatomical view of the ultrasound image.

**2.** The computer-implemented method of claim 1, further comprising controlling a user interface to provide a visual representation of the ultrasound image and each historic ultrasound image.

**3.** The computer-implemented method of claim 2, wherein the display of each historic ultrasound image is interactive.

**4.** The computer-implemented method of claim 3, further comprising, responsive to a user interaction with the interactive visual representation of any historic ultrasound image,
processing the historic ultrasound image associated with the interacted visual representation to determine one or more biometric measurements of one or more anatomical features represented in the historic ultrasound image.

**6.** The computer-implemented method of claim 5, further comprising controlling the user interface to display the determined one or more biometric measurements.

**7.** The computer-implemented method of any of claims 1 to 6, wherein each set of one or more historic ultrasound images comprises at least two historic ultrasound images.

**8.** The computer-implemented method of any of claims 1 to 7, wherein the step of processing the ultrasound image to predict the anatomical view of the ultrasound image comprises processing the ultrasound image using one or more classification algorithms.

**9.** The computer-implemented method of claim 8, wherein the one or more classification algorithms comprises one or more machine-learning classification algorithms.

**10.** The computer-implemented method of claim 9, wherein the one or more machine-learning classification algorithms comprises one or more you-only-look-once object detection algorithms.

**11.** The computer-implemented method of any of claims 1 to 10, wherein the step of predicting whether or not the ultrasound image is of a higher quality than at least one historic ultrasound image comprises performing a pairwise comparison of the ultrasound image to at least one of the one or more historic ultrasound images.

**12.** The computer-implemented method of claim 11, wherein the pairwise comparison is performed using a machine-learning comparative classification algorithm.

**13.** A computer program product comprising computer program code means which, when executed on a computing device having a processing system, cause the processing system to perform all of the steps of the method according to any of claims 1 to 12.

**14.** A processing system for tracking one or more ultrasound images of a subject, the processing system being configured to carry out all of the steps of the method according to any of claims 1 to 12.

**15.** An ultrasound imaging system comprising:
the processing system of claim 14; and
an ultrasound transducer system configured to generate the ultrasound image and each historic ultrasound image.
